(19) Europäisches Patentamt
European Patent Office
Office européen des brevets

(11) Veröffentlichungsnummer: **0 111 719**
**B1**

(12) **EUROPÄISCHE PATENTSCHRIFT**

(45) Veröffentlichungstag der Patentschrift: **08.04.87**

(21) Anmeldenummer: **83111084.6**

(22) Anmeldetag: **07.11.83**

(51) Int. Cl.⁴: **C 07 C 121/43,** C 07 C 121/66, C 07 C 121/00

# (54) Neue alpha-Iminodiacetonitrile und Verfahren zu ihrer Herstellung.

(30) Priorität: **15.11.82 DE 3242193**

(43) Veröffentlichungstag der Anmeldung: **27.06.84 Patentblatt 84/26**

(45) Bekanntmachung des Hinweises auf die Patenterteilung: **08.04.87 Patentblatt 87/15**

(84) Benannte Vertragsstaaten: **BE CH DE FR GB IT LI**

(56) Entgegenhaltungen: **CHEMICAL ABSTRACTS SERVICE REGISTRY HANDBOOK, number section 1965-1971, Band R4, Seite 7775r, Columbus, Ohio, USA**

(73) Patentinhaber: **BASF Aktiengesellschaft, Carl-Bosch- Strasse 38, D-6700 Ludwigshafen (DE)**

(72) Erfinder: **Brunnmueller, Fritz, Dr., Alsenzstrasse 7, D-6703 Limburgerhof (DE)**
Erfinder: **Kroener, Michael, Dr., Eislebener Weg 8, D-6800 Mannheim 42 (DE)**

**EP 0 111 719 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft asymmetrische α-Iminodiacetonitrile und Verfahren zu ihrer Herstellung durch Umsetzung von Aldehydcyanhydrinen mit Aminonitrilen.

Es ist aus den Annalen, Band 177 (1875), Seiten 116-139, bekannt, durch Umsetzung von Önanthaldehydammoniak mit Blausäure bei 15-47°C und Behandlung des Reaktionsprodukts mit Salzsäure das α-Iminodicaprylsäurenitril

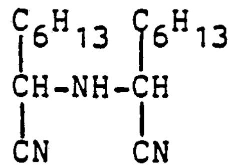

herzustellen. In ähnlicher Weise erhält man auch das α -Iminodiisovaleronitril ($R^1$, $R^2$ = Isopropyl) (Berichte, Band 13 (1880), Seiten 905-908), das α-Iminodipropionitril ($R^1$, $R^2$ = Methyl) (Annalen Band 200, Seiten 126, 127 (1880), das α-Iminodiisocapronitril ($R^1$, $R^2$ = Isobutyl) (Berichte, Band 14 (1881), Seiten 1867-1870), das α-Iminodi-(phenylpropionitril) ($R^1$, $R^2$ = Benzyl) (Annalen, Band 219, (1883), Seiten 188-194) und unter Verwendung von Hexamethylentetramin anstelle des Aldehyd-ammoniaks das unsubstituierte Iminodiacetonitril ($R^1$, $R^2$ = Wasserstoff) (Annalen, Band 278 (1894), Seiten 229-233). Von allen diesen Veröffentlichungen werden nur symmetrische Dinitrilverbindungen beschrieben.

M. Bejaud et al. (Bull. Soc- Chem- France 1976, Seiten 1425-1430) konnten zeigen daß es sich beim System Acetaldehyd/Blausäure/Methylamin (bzw. Ammoniak) um sehr komplexe Gleichgewichte zwischen Acetaldehyd-cyanhydrin, α -Aminopropionsäurenitril (bzw. der N-Methylverbindung) und X-Iminodipropionsäure-nitril (bzw. der N-Methylverbindung) untereinander und zwischen diesen Stoffen und den jeweiligen Ausgangsstoffen ihrer Bildung handelt. Wegen der leichten Einstellung dieser Gleichgewichte war es so nicht zu erwarten, daß es geligen könnte, asymmetrische Dinitrile 1 ($R^1$, $R^2$) durch gezielte Kondensation eines Aminonitrils III mit einem Aldehydcyanhydrin II herzustellen. In dieser Veröffentlichung wurde nur die Herstellung der beiden symmetrischen α -Imino- bzw. α-Methylimino-dipropionsäurenitrile gezeigt.

Es wurden nun die neuen α-Iminodiacetonitrile der Formel I,

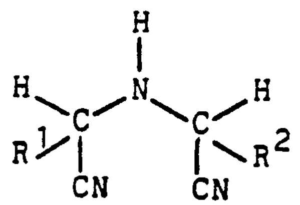

I,

worin die einzelnen Reste $R^1$ und $R^2$ verschieden sind und jeweils ein Wasserstoffatom, einen aliphatischen, cycloaliphatischen, araliphatischen oder aromatischen Rest bedeuten, gefunden.

Weiterhin wurde gefunden, daß man asymmetrische α - Iminodiacetonitrile der Formel I,

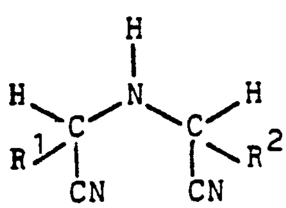

I,

worin die einzelnen Reste $R^1$ und $R^2$ verschieden sind und jeweils ein Wasserstoffatom, einen aliphatischen, cycloaliphatischen, araliphatischen oder aromatischen Rest bedeuten, durch Umsetzung von Aldehydcyanhydrinen mit Aminonitrilen vorteilhaft erhält, wenn man Aldehydcyanhydrine der Formel II,

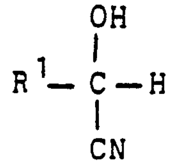

II,

worin $R^1$ die vorgenannte Bedeutung besitst, mit Aminonitrilen der Formel III,

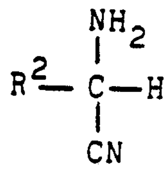

III,

worin $R^2$ die vorgenannte, von $R^1$ unterschiedliche Bedeutung besitzt, umsetzt.

Die Umsetzung kann für den Fall der Verwendung von Acetaldehydcyanhydrin und α-Aminoacetonitril durch die folgenden Formeln wiedergegeben werden:

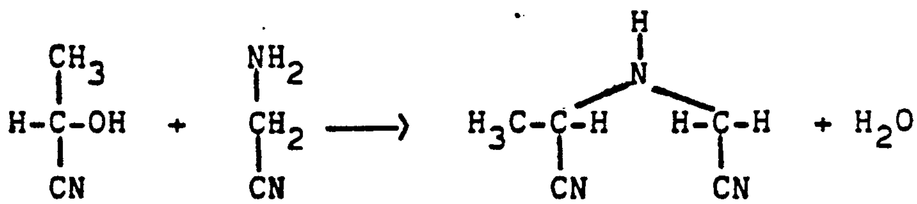

Im Hinblick auf den Stand der Technik liefert das Verfahren nach der Erfindung überraschend auf einfachem und wirtschaftlichem Wege unsymmetrische α-Iminodiacetonitrile in hoher Raumzeitausbeute, hoher Ausbeute und Reinheit. Im Hinblick auf die Veröffentlichung von Bejaud (loc.cit.) hätte man nur die Bildung von symmetrischen Endstoffen oder bestenfalls ein Gemisch heterogener, den komplexen Gleichgewichtsbedingungen entsprechend zahlreicher Endprodukte, worin α-Iminodiacetonitrile I lediglich als Nebenstoffe auftreten, erwartet. Es ist auch überraschend, daß gerade die stabilen Aminonitrile III, anstelle von Selbstkondensation eine hohe, selektive Ausbeute an Endstoff I liefern.

Weiterhin ist es überraschend, daß man als Ausgangsstoffe III rohe, wasserhaltige Aminonitrile, z.B. das ungereinlgte Reaktionsprodukt einer Aminonitrllsynthese, verwenden kann, ohne daß eine wesentliche Hydrolyse des Endstoffs 1 eintritt.

In Tabelle 1 sind Abbauraten von Aminonitrilen III zu α -Iminodiacetonitrilen, worin $R^1$ und $R^2$ gleich sind und die vorgenannte Bedeutung besitzen, gezeigt. Lösungen von Aminonitrilen in der angegebenen Konzentration an Ausgangsstoff III und Lösungsmittel werden während der angegebenen Abbauzeit bei 22°C gehalten. Der Abbau wurde durch Analyse des Gehalts (alle Angaben in Gramm) verfolgt. Meist sind unter den aliphatischen Aminonitrilen die Aminonitrile mit dem jeweils höheren aliphatischen Rest stabiler, wie Tabelle 1 zeigt. Aus den Daten dieser Tabelle geht aber auch hervor, daß in Gegenwart von Wasser oder insbesondere Methanol eine hohe Neigung zur Selbstkondensation vorliegt. Die hohe Selektivität der erfindungsgemäßen Herstellung von asymmetrischen Endstoffen auch in Gegenwart dieser Lösungsmittel ist daher überraschend.

**Tabelle I**

| Zeit | $R^1,R^2$=H | | $R^1,R^2=CH_3$ | | $R^1,R^2=CH_3$ | | $R^1,R^2=CH_3$ | | $R^1,R^2=C_2H_5$ | |
|---|---|---|---|---|---|---|---|---|---|---|
| (h) | Gehalt | $H_2O$ | Gehalt | $H_2O$ | Gehalt | $H_2O$ | Gehalt | MeOH | Gehalt | $H_2O$ |
| 0 | 97,6 | 2,4 | 100 | 0 | 80 | 20 | 25 | 75 | 98 | 2 |
| 8 | | | | | 68 | | | | | |
| 24 | 95,3 | | 90 | | | | 19 | | 94 | |
| 144 | 90,5 | | 69 | | | | 13 | | 75 | |

Man kann die Ausgangsstoffe II und III in stöchiometrischer Menge oder jede der Komponenten jeweils im Uberschu59 umsetzen, vorteilhaft in einer Menge von 1 bis 2, vorzugsweise 1 bis 1,2 Mol Aldehydcyanhydrin II je Mol Aminonitril III. Bevorzugte Ausgangsstoffe II und III und dementsprechend bevorzugte Endstoffe 1 sind solche, in deren Formeln $R^1$ und $R^2$ verschieden sind und jeweils ein Wasserstoffatom, einen Alkylrest mit 1 bis 20, vorzugsweise 1 bis 8, insbesondere 1 bis 4 Kohlenstoffatomen oder einen durch Alkoxy- und/oder Alkylthiogruppen mit zweckmäßlg 1-4 Kohlenstoffatomen, Halogenatome, bevorzugt Chloratome oder Fluoratome, Carbonamidgruppen oder Cyanogruppen, insbesondere eine dieser Gruppen oder Atome substltuierten Alkylrest mit 1 bis 20, vorzugswelse 1 bis 8, insbesondere 1 bis 4 Kohlenstoffatomen, einen Alkenylrest mit 2 bis 20, vorzugsweise 2 bis 8, insbesondere 2 bis 4 Kohlenstoffatomen, einen Cycloalkylrest mit 5 bis 8 Kohlenstoffatomen, einen Aralkylrest oder Alkylarylrest mit 7 bis 12 Koblenstoffatomen oder einen Phenylrest bedeuten. Die vorgenannten Reste können noch durch unter den Reaktionsbedingungen inerte Gruppen oder Atome, z.B. Alkylgruppen, Alkoxygruppen, Alkylthiogruppen mit jeweils 1 bis 4 Kohlenstoffatomen, Carbalkoxygruppen mit 2 bis 8 Kohlenstoffatomen, substituiert sein.

Es kommen z.B. als Ausgangsstoffe II in Betracht: Das Cyanhydrin von Formaldehyd, Acetaldehyd, Propionaldehyd, n-Butyraldehyd, iso-Butyraldehyd, 2-Methyl-butyraldehyd, 2-Ethyl-capronaldehyd, n-Valeraldehyd, Isovaleraldehyd, 2,2-Dimethyl-propionaldehyd, n-Capronaldehyd, Isocapronaldehyd, 2-Methylvaleraldehyd, 3-Methyl-valeraldehyd, 2-Ethylbutyraldehyd, 2,2-Dimethylbutyraldehyd, 2,3-Dimethylbutyraldehyd, 3,3-Dimethylbutyraldehyd, Önanthaldehyd, 2-Methyl-capronaldehyd, 3-Methyl-capronaldehyd, 4-Methyl-capronaldehyd, 5-Methyl-capronaldehyd, 2-Ethyl-valeraldehyd, 2,2-Dimethylvaleraldehyd, 3-Ethylvaleraldehyd, 2,3-Dimethylvaleraldehyd, 4-Ethyl-valeraldehyd, 4,4-Dimethyl-valeraldehyd, 3,4-Dimethylvaleraldehyd, 2,4-Dimethyl-valeraldehyd, 2-Ethyl-2-methyl-butyraldehyd, 2-Ethyl-3-methylbutyraldehyd, Cyclohexylaldehyd, Benzaldehyd, Phenylacetaldehyd.

Es kommen z.B. als Ausgangsstoffe III in Betracht: Unsubstituiertes oder in 2-Stellung durch die Methyl-, Ethyl-, n-Propyl-, Isopropyl-, n-Butyl-, Isobutyl-, sek.-Butyl-, Cyclohexyl-, Benzyl-, Phenyl-gruppe substltuierte 2-Amino-acetonitrile.

Die Umsetzung wird zweckmäßig bei einer Temperatur von 0 bis 100, vorzugsweise von 20 bis 60°C, mit Unterdruck oder Überdruck oder vorzugsweise drucklos, diskontinuierlich oder kontinuierlich durchgeführt. Die Umsetzung der Ausgangsstoffe II und III findet in Gegenwart des sich bildenden Reaktionswassers statt; man kann zwar dieses Reaktionswasser kontinuierlich während der Reaktion abtrennen, doch es ist zweckmäßig und wirtschaftlicher, das Reaktionswasser erst bei der Aufarbeitung zu entfernen. Gegebenenfalls kann auch zusätzliches Wasser zur Beschleunigung der Reaktion während der Reaktion anwesend sein. Ebenfalls ist es möglich und wirtschaftlich, rohe und gegebenenfalls wasserhaltige Ausgangsstoffe zu verwenden, wie sie z.B. auch bei der Herstellung der Ausgangsstoffe anfallen. So kann z.B. das Aldehydcyanhydrin II durch Umsetzung von Blausäure mit Aldehyden hergestellt und das Reaktionsgemisch direkt für das erfindungsgemäße Reaktlonsgemisch verwendet werden. Ebenfalls kann z.B. das Aminonitril III durch Umsetzung von Ausgangsstoff II mit $NH_3$ hergestellt und aus dem Reaktionsgemisch lediglich durch Strippen das überschüssige $NH_3$ abgetrennt werden; in solchen Fällen tritt der Ausgangsstoff III mit Wasser in das erfindungsgemäße Ausgangsgemisch ein. Im allgemeinen können Wassermengen von 1 bis 2 Mol Gesamtwasser je Mol Ausgangsstoff II im Reaktionsgemisch vorhanden sein.

In Abwesenheit oder Anwesenheit von Wasser kann man bei der Herstellung von asymmetrischen Endstoffen 1 aus Ausgangsstoffen II und Ausgangsstoffen III auch niedere Alkanole zur Beschleunigung der Reaktion verwenden, zweckmäßig in einer Menge von 5 bis 95, insbesondere 25 bis 75 Gew.-% Ausgangsstoff III, bezogen auf die Gewichtsmenge an niederem Alkanol. Als niedere Alkanole kommen zweckmäßig solche mit 1 bis 6 Kohlenstoffatomen, vorteilhaft Ethanol, Propanol, Isopropanol, n-Butanol, Isobutanol, sek.-Butanol, Tert.-Butanol und insbesondere Methanol in Frage.

Die Reaktion kann wie folgt durchgeführt werden: Ein Gemisch von Ausgangsstoff II und III gegebenenfalls zusammen mit Wasser und/oder einem Alkanol, zweckmäßig in vorgenannten Mengen, wird während der Reaktionszeit, zweckmäßig von 8 bis 100 Stunden, bei der Reaktionstemperatur gehalten. Dann wird aus dem Reaktionsgemisch der Endstoff in üblicher Weise, z.B. durch Destillation bzw. Entfernung des Wassers, isoliert. Der Endstoff ist in der Regel schon so rein, daß er direkt weiterverarbeitet werden kann. So braucht man lediglich das Reaktionswasser im Vakuum abdestillieren. Die häufigsten Verunreinigungen sind nicht umgesetzte Aminonitrile oder Cyanhydrine, die extraktiv oder destillativ, besonders schonend durch eine Dünnschichtverdampfung oder - sofern sie genügend wasserlöslich sind - durch eine Wasserwäsche ganz oder teilweise entfernt werden können. Man kann aber auch Aminonitril III und Dinitril I in einem wasserfreien Lösungsmittel z.B. als Hydrochloride fällen und beide Salze mit Wasser behandeln. Dabei wird das Dinitrilsalz hydrolytisch gespalten, während das stärker basische Aminonitril III als wasserlösliches Salz abgetrennt werden kann.

Die nach dem Verfahren der Erfindung erhältlichen $\alpha$ -Iminodiacetonitrile sind wertvolle Ausgangsstoffe für die Herstellung von Farbstoffen, Fungiciden, Bactericiden, Herbiciden, Textilhilfsmitteln, Korrosionsschutzmitteln, Komplexbildern in Galvanisierbädern und Inhibitoren von Frostschutzmitteln. Bezüglich der Verwendung wird auf die vorgenannten Veröffentlichungen verwiesen.

In einer bevorzugten Verwendungsform werden aus den so erhaltenen Endstoffen 1 nach den in den deutschen Patentanmeldungen P 32 42 195.8 und P 32 42 266.0 beschriebenen Arbeitsweisen zunächst 2-Aminopyrazine der Formel Ia

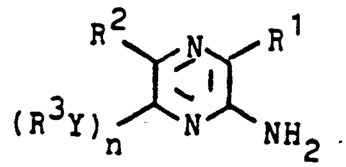

Ia

durch Umsetzung

1a) mit Halogenwasserstoffen der Formel XI,

H - X XI,

oder

1b) mit Alkoholen und/oder Thioalkoholen der Formel VII,

$HYR^3$ VII,

und Halogenwasserstoffen der Formel XI

H - X XI,

oder

1c) mit Alkoholen und/oder Thioalkoholen der Formel VII

- $HYR^3$ VII

in Gegenwart von Alkali- und/oder Erdalkaliverbindungen, hergestellt.

Die Reste $R^1$, $R^2$ und X haben die vorgenannten allgemeinen und bevorzugten Bedeutungen. $R^3$ hat die allgemeinen und bevorzugten Bedeutungen von $R^1$ und $R^2$, bezeichnet aber nicht ein Wasserstoffatom. Y steht für ein Sauerstoffatom oder Schwefelatom und n bezeichnet 0 oder, wenn die Umsetzung 1c) durchgeführt wird, gegebenenfalls 1. Man kann die Stoffe in stöchiometrischer Menge oder jede der Komponenten jeweils im Überschuß zur anderen umsetzen, vorzugsweise in einem Verhältnis im Falle des Verfahrens 1a) von 1,5 bis 6, insbesondere 2 bis 5 Mol Ausgangsstoff XI je Mol Stoff I, im Falle des Verfahrens 1b) von 1,5 bis 6, insbesondere 2 bis 5 Mol Ausgangsstoff XI und/oder 0,1 bis 10, insbesondere 0,5 bis 3 Mol Ausgangsstoff VII je Mol Stoff I, im Falle des Verfahrens 1c) von 5 bis 50, insbesondere 10 bis 25 Gew.-% Stoff I je Gewichtsmenge Stoff VII. Die Umsetzung (1a, 1b, 1c) wird vorteilhaft bei einer Temperatur von 0 bis 100, im Falle der Umsetzung 1a) zweckmäßig von 40 bis 80°C, im Falle der Umsetzung 1b) zweckmäßig von 20 bis 80, bevorzugt von 30 bis 70°C, im Falle der Umsetzung 1c) zweckmäßig von 0 bis 80, bevorzugt 20 bis 60°C, mit Unterdruck oder Überdruck oder drucklos, diskontinuierlich oder kontinuierlich durchgeführt. Die Reaktionszeit beträgt zweckmäßig 0,1 bis 200, vorzugsweise 3 bis 48 Stunden. Man verwendet gegebenenfalls unter den Reaktionsbedingungen inerte, organische Lösungsmittel. Als Lösungsmittel kommen z.B. in Frage; aromatische, aliphatische oder cycloaliphatische Kohlenwasserstoffe; Halogenkohlenwasserstoffe, insbesondere Chlorkohlenwasserstoffe; Ether, Mercaptane. Zweckmäßig verwendet man das Lösungsmittel in einer Menge von 40 bis 10 000 Gewichtsprozent, vorzugsweise von 50 bis 1 500 Gewichtsprozent, bezogen auf Stoff I. Man kann gegebenenfalls auch den Ausgangsstoff VII selbst als Lösungsmittel nebmen.

Die Umsetzung 1c) wird in Gegenwart von Erdalkaliverbindungen oder insbesondere Alkaliverbindungen, vorteilhaft in katalytischen Mengen, zweckmäßig in einer Menge von 0,1 bis 2, vorzugsweise 0,2 bis 0,8 Äquivalenten Base, bezogen auf ein Mol Stoff I, durchgeführt. Vorteilhaft werden Alkoholate, Mercaptide, Hydroxide oder Cyanide sowie entsprechende Gemische verwendet.

Aus den so erhaltenen Stoffen Ia, in reiner oder roher Form, werden bevorzugt 2-Halogen- oder 2Cyanpyrazine der Formel

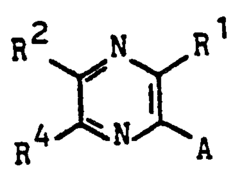

Ic

durch Umsetzung bei einer Temperatur von -50 bis +50°C,

2a) mit Alkalinitriten oder Alkylnitriten in Gegenwart von Wasser und/oder organischen Lösungsmitteln 2a1) mit Tetrafluorborsäure oder 2a2) Halogenwasserstoffsäuren der Formel

H - X XI,

in Form einer 10 bis 80 gewichtsprozentigen Lösung und mit einer Menge von 1 bis 5 Mol Ausgangsstoff XI je Mol Stoff Ia oder

2b) mit Nitrosylhalogeniden in Gegenwart von unter den Reaktionsbedingungen inerten organischen Lösungsmitteln

und gewünschtenfalls durch Umsetzung der so erhaltenen 2-Halogenpyrazine der Formel

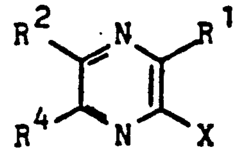

VIII

mit Kupfercyanid und gegebenenfalls Alkali- und/oder Erdalkalicyaniden bei einer Temperatur von 80 bis 200°C hergestellt. Die Reste $R^1$, $R^2$, $R^3$, Y und X haben die vorgenannte allgemeine und bevorzugte Bedeutung, wobei A ein Halogenatom, vorzugsweise ein Chloratom, Bromatom oder Fluoratom oder eine Cyangruppe und $R^4$ ein Wasserstoffatom oder den Rest $R^3$Y- bedeuten.

Bevorzugte Ausgangsstoffe XI sind Chlor- und Bromwasserstoffsäuren. Als Nitrosylhalogenide werden solche der Formel

O = N - X IX,

verwendet. Bevorzugte Alkalinitrite sind Natrium- und Kaliumnitrit. Bevorzugte Alkylnitrite sind solche der Formel

O = N - O - $R^5$ X,

worin $R^5$ einen Alkylrest mit 1 bis 6 Kohlenstoffatomen bedeutet, zweckmäßig Amylnitrit, Ethylnitrit, Neopentylnitrit. Verfahren 2a1 (Tetrafluorborwasserstoffsäure) wird zweckmäßig mit organischen Lösungsmitteln oder bevorzugt mit Gemischen von zusätzlichem Wasser und organischen Lösungsmitteln durchgeführt. Verfahren 2a2 kann mit organischen Lösungsmitteln und gegebenenfalls im Gemisch mit zusätzlichem Wasser, zweckmäßig aber in wäßrigem Medium durchgeführt werden.

Anstelle der Ausgangsstoffe IX kann man auch die Reaktionsgemische ihrer Herstellung, z.B. aus NO und Halogen, insbesondere Brom, verwenden. Mann kann die Ausgangsstoffe in stöchiometrischer Menge oder jede der Komponenten jeweils im Überschuß zur anderen umsetzen, vorzugsweise in einem Verhältnis von 1 bis 3, insbesondere von 1,05 bis 1,2 Mol Alkalinitrit oder Alkylnitrit, vorzugsweise 1 bis 3, insbesondere von 1,05 bis 1,2 Mol $HBF_4$ oder von 1 bis 3, insbesondere 1,05 bis 1,5 Mol Ausgangsstoff IX je Mol Stoff 1a. Man verwendet 1 bis 5, vorzugsweise 2 bis 4 Mol Ausgangsstoff XI je Mol Stoff 1a. Die Umsetzung 2a) bzw. 2b) wird bei einer Temperatur von -50 bis +50°C, im Falle des Verfahrens 2a) zweckmäßig von -30 bis +40, insbesondere von -20 bis +25°C, im Falle des Verfahrens 2b) zweckmäßig von -25 bis +40, insbesondere von -25 bis 0°C, mit Unterdruck oder Überdruck oder drucklos, diskontinuierlich oder kontinuierlich durchgeführt. Man verwendet für Verfahren 2a) und 2b) Lösungsmittel. Wasser wird ganz oder teilweise in Gestalt von Lösungen der Säuren XI und vorteilhaft in Gestalt von Lösungen des Alkalinitrits der Reaktion zugeführt. Als organische Lösungsmittel kommen im allgemeinen je nach Verfahren 2a) oder Verfahren 2b) in Frage:. Halogenkohlenwasserstoffe (bevorzugt 2b), insbesondere Chlorkohlenwasserstoffe; Ether (bevorzugt 2a); Alkanole und Cycloalkanole (bevorzugt 2a); Sulfoxide und Sulfone; Ester (bevorzugt in Abwesenheit zusätzlicher Wassermengen); Carbonsäuren (bevorzugt 2a) mit 2 bis 6 Kohlenstoffatomen; und entsprechende Gemische. Zweckmäßig verwendet man das organische Lösungsmittel und/oder zusätzliches Wasser (ohne Reaktionswasser) in einer Menge von 50 bis 5000 Gewichtsprozent, vorzugsweise 100 bis 1000 Gewichtsprozent, bezogen auf Stoff 1a.

Man kann einen Teil des Lösungsmittels oder die Gesamtmenge auch in Gestalt der entsprechenden Lösung der Ausgangsstoffe, z.B. der Tetrafluorborsäure, einsetzen. Ausgangsstoff XI wird in Form einer 10 bis 80, vorzugsweise 30 bis 70 gewichtsprozentigen Lösung verwendet. Die Reaktionszeit beträgt zweckmäßig 0,2 bis 5 Stunden. In einer bevorzugten Ausführungsform arbeitet man mit Nitrosylbromid im Eintopfverfahren. Zunächst wird das in einem geeigneten Lösungsmittel, z.B. einem der vorgenannten, vorgelegte Brom durch Einleiten von NO bei +10°C bis -40°C, vorzugsweise -10°C bis -20°C, in NOBr überführt und anschließend das in einem vorgenannten Lösungsmittel gelöste 2-Aminopyrazin 1a bei der gleichen Temperatur zudosiert.

Der Stoff VIII kann mit Kupfercyanid allein in stöchiometrischer Menge oder zweckmäßig im Überschuß, vorteilhaft in einer Menge von 1 bis 2, bevorzugt 1,05 bis 1,5 Mol Kupfercyanid je Mol Ausgangsstoff VIII umgesetzt werden. Anstelle eines Anteils an Kupfercyanid können Alkali- und/oder Erdalkalicyanide, zweckmäßig Lithiumcyanid, Kalziumcyanid, Bariumcyanid, bevorzugt Natriumcyanid oder Kaliumcyanid, verwendet werden, zweckmäßig in Mengen von 1 bis 3 Mol, vorzugsweise 1,1 bis 1,5 Mol Alkali- und/oder Erdalkalicyanide je Mol Ausgangsstoff VIII. Vorteilhaft sind Gemische von 10 bis 50, bevorzugt 15 bis 25 Mol Kupfercyanid, bezogen auf 100 Mol % aller Cyanide. Die Umsetzung wird bei einer Temperatur von 80 bis 200°C vorzugsweise 120 bis 160°C, drucklos, unter Überdruck oder Unterdruck, kontinuierlich oder diskontinuierlich durchgeführt. Man verwendet zweckmäßig unter den Reaktionsbedingungen inerte heterocyclische Lösungsmittel, zweckmäßig in einer Menge von 100 bis 5000, bevorzugt 200 bis 1000 Gewichtsprozent Lösungsmittel, bezogen auf Stoff Ia. Die Reaktionszeit beträgt 1 bis 12 Stunden.

Durch diese elegante Arbeitsweise in 3 Stufen (III → I → Ia → Ic) werden zahlreiche Synthesewege auf den vorgenannten Industriegebieten, z.B. auch für Folsäureverbindungen, überraschend ermöglicht bzw. wirtschaftlicher und einfacher.

**Beispiele 1-19**

Molare Mengen Cyanhydrin II und Aminonitril III wurden unter den in Tabellen 1 und 3 angegebenen Bedingungen umgesetzt. Der Fortgang der Reaktion wurde potentiometrisch (Umsetzung des Aminonitrils III) und dünnschichtchromatographisch verfolgt. War der Umsatz konstant, wurde im Vakuum bei der Reaktionstemperatur das Reaktionswasser und gegebenenfalls das Lösungsmittel abdestilliert (Aufarbeitung I). Der nach Entfernung des Lösungsmittels verbleibende Rückstand kann destilliert werden (Aufarbeitung 1a). Größere Reaktionsgemische wurden zweistufig in einem Dünnschichtverdampfer bei Temperaturen von 20 bis 200°C und Verweilzeiten von 0,1 bis 10 Minuten im Vakuum entwässert (Aufarbeitung II) und im Bedarfsfall auch destilliert (IIa). Zur Struktursicherung wurden Elementaranalysen und $H^1$- bzw. $C^{13}$-NMR-Analysen durchgeführt. Die Reinheitskontrolle erfolgte über Dünnschichtchromatographie, kombiniert mit potentiometrischer Titration des nicht umgesetzten Aminonitrils und einer Cyanhydrinbestimmung nach Liebig. Einzelheiten sind den Tabellen 2, 2a und 3 zu entnehmen. Die Zusammensetzung der Reaktionsgemische in Tabelle 2a ist in Gew.-% angegeben.

**Tabelle 2**

(Herstellung asymmetrischer α-Iminodiacetonitrile)

| Beisp. | Cyanhydrin $R^1$ | Menge in g | Aminonitril $R^2$ | Menge in g | $H_2O$ (g) | Temp. (°C) | Zeit (d) | Methanol (g) | Umsatz (%) | Selektivität (%) |
|---|---|---|---|---|---|---|---|---|---|---|
| 1 | $CH_3$ | 142 | H | 112 | 3 | 40 | 1 | 257 | 95 | 97 |
| 1a | $CH_3$ | 284 | H | 224 | 27 | 40 | 1 | - | 97 | 99 |
| 2 | $C_2H_5$ | 255 | H | 168 | 5 | 25 | 3 | - | 94 | 97 |
| 3 | i-$C_3H_7$ | 198 | H | 112 | 3 | 25 | 3 | - | 93 | 95,4 |
| 4 | i-$C_4H_9$ | 452 | H | 224 | 7 | 25 | 3 | - | 91 | 98 |
| 4a | i-$C_4H_9$ | 452 | H | 224 | 27 | 50 | 0,4 | - | 96 | 99 |
| 5 | $MeOCH_2$ | 10,1 | H | 5,6 | - | 50 | 3 | 17 | 92 | 94 |
| 6 | $CH_3$ | 284 | $C_2H_5$ | 336 | 17 | 25 | 2 | - | 96 | 88 |
| 7 | $CH_3$ | 284 | i-$C_3H_7$ | 392 | 55 | 40 | 2,5 | - | 92 | 93 |
| 8 | $CH_3$ | 7 | $CH_3SCH_2CH_2$ | 13 | 0,2 | 40 | 3 | - | 93 | 94 |
| 9 | $CH_3$ | 71 | $CH_3OCH_2$ | 100 | 2,4 | 40 | 2,5 | 185 | 94,5 | 94 |
| 10 | $CH_3$ | 35,5 | $C_2H_5OCH_2$ | 57 | 0,3 | 40 | 4 | - | 94,5 | 96 |
| 11 | $CH_3$ | 142 | $C_2H_5$-CH-$C_4H_9$ | 308 | 3 | 40 | 1 | - | 97 | 95 |
| 12 | $CH_3$ | 57 | $C_7H_7$ | 117 | 1 | 50 | 3 | - | 96 | 93 |
| 13 | $MeOCH_2$ | 10,1 | $C_2H_5OCH_2$ | 11,4 | - | 50 | 3 | 23 | 95 | 90 |
| 14 | $MeOCH_2$ | 20,2 | i-$C_3H_7$ | 19,6 | 3 | 50 | 4 | 47 | 94,5 | 87 |
| 15 | $CH_3$ | 14 | $C_6H_5$ | 26,4 | - | 40 | 4 | - | 90 | 75 |

**Tabelle 2a**

(Qualität der asymmetrisch substituierten α-Iminodiacetonitrile in Gew.-%)

1) 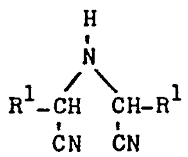

2) 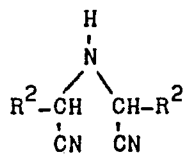

3) 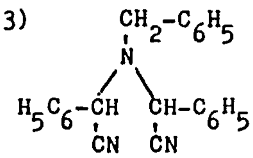

| Beispiel | aufgearbeitet nach Verfahren | Endstoff I | Ausgangsstoff III | Ausgangsstoff II | Nebenstoff 1) | Nebenstoff 2) (3) | $n_D^{20}$ (Fp) |
|---|---|---|---|---|---|---|---|
| 1 | IIa | 94 | 1,3 | 1,7 | 1,5 | 1,5 | 1.4511 |
| 1a | II | 97,8 | 1,0 | 1,0 | Spur | Spur | 1.4492 |
| 2 | I | 90 | 1,1 | | | | |
| | Ia | 95 | 0,8 | | 1 | 1 | 1.4506 |
| 3 | I | 92 | 2,5 | | 1 | 0,5 | |
| 4 | I | 92 | 2,7 | | 1 | 1 | |
| 4a | II | 95,5 | 2 | 0,5 | 1 | 1 | 1.4530 |
| 5 | I | 90 | 1,9 | | | 3 | |
| 6 | II | 92 | 1,1 | 1,9 | 3 | 2 | 1.4455 |
| 7 | I | 92 | 5 | | 2 | 1 | |
| 8 | I | 89 | 2,6 | | 3 | | |

**Tabelle 2a** (Fortsetzung)

| Beispiel | aufgearbeitet nach Verfahren | Endstoff I | Ausgangsstoff III | Ausgangsstoff II | Nebenstoff 1) | Nebenstoff 2) (3) | $n_D^{20}$ (Fp) |
|---|---|---|---|---|---|---|---|
| 9 | IIa | 94 | 4 | | 2 | | 1.4492 |
| 10 | I | 94 | 3 | | | 1 | |
| 11 | II | 92 | 3 | | 2 | 3 | |
| 12 | I | 90 | 3 | | 5 | 2 | (Fp.40°C) |
| 13 | I | 87 | 3 | | 3 | 7 | |
| 14 | I | 84,5 | 2,5 | | 10 | 3 | |
| 15 | I | 74 | 4 | | 17 | (5) | |

**Patentansprüche**

1. α-Iminodiäcetonitrile der Formel I,

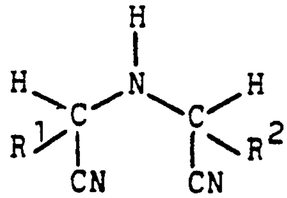

I,

worin die einzelnen Reste $R^1$ und $R^2$ verschieden sind und jeweils ein Wasserstoffatom, einen aliphatischen, cycloaliphatischen, araliphatischen oder aromatischen Rest bedeuten.

2. Verfahren zur Herstellung von asymmetrischen α-Iminodiacetonitrilen der Formel 1,

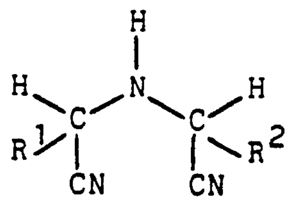

I,

worin die einzelnen Reste $R^1$ und $R^2$ verschieden sind und jeweils ein Wasserstoffatom, einen aliphatischen, cycloaliphatischen, araliphatischen oder aromatischen Rest bedeuten, durch Umsetzung von Aldehydcyanhydrinen mit Aminonitrilen, dadurch gekennzeichnet, daß man Aldehydcyanhydrine der Formel II,

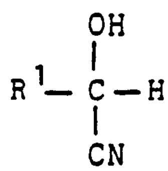

II,

worin $R^1$ die vorgenannte Bedeutung besitzt, mit Aminonitrilen der Formel III,

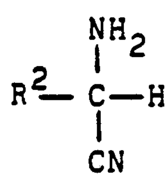

III,

worin $R^2$ die vorgenannte, von $R^1$ unterschiedliche Bedeutung besitzt, umsetzt.

**Claims**

1. An α-iminodiacetonitrile of the formula I

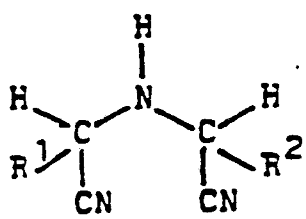

I,

where the individual radicals $R^1$ and $R^2$ are different and are each hydrogen or an aliphatic, cycloaliphatic, araliphatic or aromatic radical.

2. A proccss for the preparation of an asymmetric α-iminodiacetonitrile of the formula I

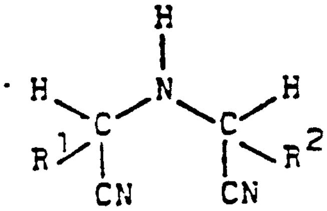

I ,

where the individual radicals $R^1$ and $R^2$ are different and are each hydrogen or an aliphatic, cycloaliphatic, araliphatic or aromatic radical, by reacting an aldehyde-cyanohydrin with an aminonitrile, wherein an aldehyde-cyanohydrin of the formula II

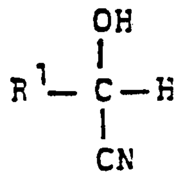

II,

where $R^1$ has the above meanings, is reacted with an aminonitrile of the formula III

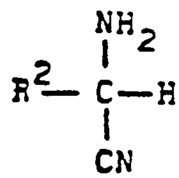

III,

where $R^2$ has one of the above meanings which is different from that of $R^1$.

**Revendications**

1. α-iminodiacetonitriles de formule I

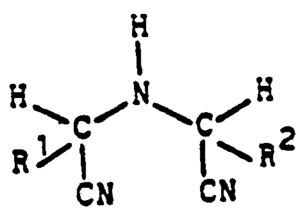

I,

dans laquelle les différents radicaux $R^1$ et $R^2$ sont dissemblables et représentent chacun un atome d'hydrogène, un radical aliphatique, cycloaliphatique, araliphatique ou aromatique.

2. Procédé de préparation d'α-iminodiacétonitriles asymétriques de formule I

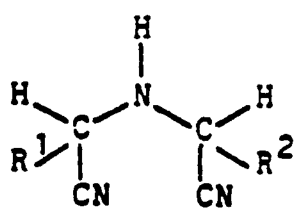

I,

dans laquelle les différents radicaux $R^1$ et $R^2$ sont dissemblables et représentent chacun un atome d'hydrogène, un radical aliphatique, cycloaliphatique, araliphatique ou aromatique, par réaction d'aldéhyde-cyanhydrines avec des aminonitriles, caractérisé en ce qu'on fait réagir des aldéhyde-cyanhydrines de formule II

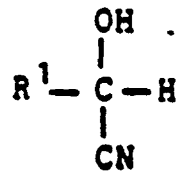

II,

dans laquelle $R^1$ a la signification donnée ci-dessus, avec des aminonitriles de formule III

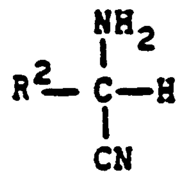

III,

dans laquelle $R^2$ a la signification donnée ci-dessus, différente de la signification de $R^1$.